(19) Europäisches Patentamt
European Patent Office
Office européen des brevets

(11) Veröffentlichungsnummer : **0 214 423**
**B1**

(12) **EUROPÄISCHE PATENTSCHRIFT**

(45) Veröffentlichungstag der Patentschrift : **03.01.90**

(51) Int. Cl.$^5$ : **C 07 D277/64,** C 07 D277/66

(21) Anmeldenummer : **86110100.4**

(22) Anmeldetag : **23.07.86**

(54) **Verfahren zur Herstellung 2-substituierter Benzthiazole.**

(30) Priorität : **05.08.85 DE 3528032**

(43) Veröffentlichungstag der Anmeldung : **18.03.87 Patentblatt 87/12**

(45) Bekanntmachung des Hinweises auf die Patenterteilung : **03.01.90 Patentblatt 90/01**

(84) Benannte Vertragsstaaten : **CH DE FR GB IT LI**

(56) Entgegenhaltungen :
**EP-A- 0 039 483**
**EP-A- 0 116 827**
**US-A- 3 102 142**

(73) Patentinhaber : **HOECHST AKTIENGESELLSCHAFT**
**Postfach 80 03 20**
**D-6230 Frankfurt am Main 80 (DE)**

(72) Erfinder : **Papenfuhs, Theodor, Dr.**
**Heinrich-Bleicher-Strasse 40**
**D-6000 Frankfurt-am-Main 50 (DE)**

**EP 0 214 423 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Gegenstand der Erfindung ist ein hinsichtlich Gewerbehygiene und Abwasserentsorgung und hinsichtlich höherer Ausbeute verbessertes Verfahren zur Herstellung von substituierten Benzthiazolen, die wertvolle Zwischenprodukte zur Herstellung von Farbstoffen, Pflanzenschutzmitteln und Pharmazeutika sind.

2-Substituierte Benzthiazole der allgemeinen Formel (1)

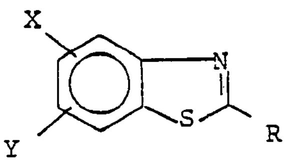

(1)

in welcher R eine Alkyl($C_1$-$C_6$) oder Alkenyl($C_2$-$C_6$)-gruppe, welche durch Alkoxy($C_1$-$C_4$)-gruppen, Acylgruppen, wie beispielsweise Acetyl- oder Benzoylgruppen, eine Phenyl-, Chlorphenyl-, Bromphenyl-, Alkyl($C_1$-$C_6$)-phenylgruppe, wie beispielsweise Methylphenyl- oder Ethylphenylgruppen, eine Alkoxy($C_1$-$C_4$)-phenylgruppe, wie beispielsweise Methoxyphenyl- oder Ethoxyphenylgruppen, oder eine Nitrophenylgruppe oder Halogenatome, wie beispielsweise Chlor- oder Bromatome, substituiert sein können, oder eine Phenylgruppe, die durch Alkyl($C_1$-$C_4$)-, Alkoxy($C_1$-$C_4$), Carboxyl-, —COO—Alkyl-, Cyan- oder Nitrogruppen oder durch Halogenatome, wie beispielsweise Chlor- oder Bromatome, substituiert sein kann, bedeutet, X und Y je ein Wasserstoff- oder Halogenatom, beispielsweise ein Chlor- oder Bromatom, oder eine Alkyl($C_1$-$C_4$), Alkoxy($C_1$-$C_4$)- oder Nitrogruppe oder zusammen einen anellierten Benzolring bedeuten, konnten bisher technisch hergestellt werden durch Umsetzung von o-Aminothiophenolen der allgemeinen Formel (2)

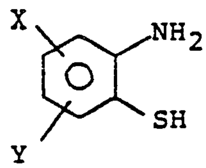

(2)

in welcher X und Y die vorstehend genannten Bedeutungen haben, mit Verbindungen der allgemeinen Formel (3) (Carbonsäuren, Carbonsäurehalogeniden oder Carbonsäureanhydriden)

$$R—CO—Z \qquad (3)$$

in welcher R die vorstehend genannte Bedeutung hat und Z ein Halogenatom, beispielsweise ein Chlor- oder Bromatom, eine Hydroxylgruppe oder die Gruppe

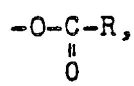

worin R die weiter oben genannte Bedeutung hat, bedeuten, gemäß dem Reaktionsschema

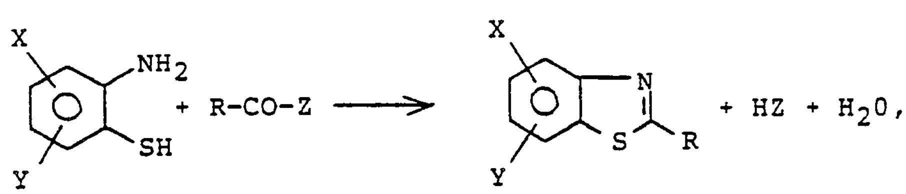


oder mit speziellen Carbonsäureanhydriden, wie beispielsweise Diketen.

Die als Ausgangsprodukte eingesetzten o-Aminothiophenole werden dabei aus entsprechenden Nitrovorstufen, in Einzelfällen auch durch alkalische Totalhydrolyse von 2-Aminobenzthiazolen oder Benzthiazthioniumhalogeniden hergestellt.

Alle bekannten technischen Synthesen von 2-substituierten Benzthiazolen der genannten Formel (1) laufen somit über freie Arylmercaptane ab, wodurch sich erhebliche Probleme hinsichtlich Gewerbehygiene und Abwasserentsorgung ergeben.

Außerdem bestand auch aus chemischer Sicht aus folgendem Grund ein Bedürfnis nach einem

effektiveren Verfahren zur technisch praktikablen Herstellung der Verbindungen der Formel (1):

Die Ausgangsprodukte der genannten Formel (2) sind als freie Mercaptane sehr oxidationsempfindlich und machen durch Übergang in 2-Aminophenyldisulfide Ausbeuteminderungen unvermeidbar.

Es wurde nun überraschenderweise gefunden, daß man 2-substituierte Benzthiazole der weiter oben genannten allgemeinen Formel (1) unter vollständiger Vermeidung der den bekannten Herstellungsverfahren anhaftenden, vorstehend erwähnten Nachteile herstellen kann, indem man die in bekannter Weise [EP-PS 0 039 483 (US-PS 4 370 483)] durch Umsetzung von 2-Aminobenzthiazolen der allgemeinen Formel (4)

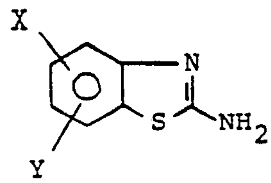

(4)

in welcher X und Y die vorstehend genannten Bedeutungen haben, mit Alkalimetall- oder Erdalkalimetallhydroxiden in einem wasserfreien oder praktisch wasserfreien Lösemittel wie Ethanol, Isobutanol, 1,2-Dihydroxypropan oder 1,3-Dihydroxypropan, bevorzugt Ethylenglykol, Glycerin, Ethylenglykol-monomethylether oder Ethylenglykol-monoethyläther, als Zwischenverbindungen in hohen Ausbeuten erhältlichen Salze der entsprechenden 2-Mercaptophenylharnstoffe der allgemeinen Formel (5)

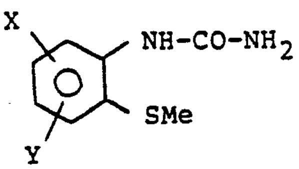

(5)

in welcher X und Y die genannten Bedeutungen haben, und Me ein Alkalimetallatom oder die äquivalente Menge eines Erdalkalimetallatoms bedeutet, in isolierter Form oder in der im Ringöffnungsgemisch vorliegenden Suspension mit einem Acylierungsmittel der allgemeinen Formel (3)

$$R\text{—}CO\text{—}Z \qquad (3)$$

in welcher R und Z die weiter oben genannten Bedeutungen haben, oder mit einem speziellen Carbonsäureanhydrid, wie beispielsweise Diketen, bei Temperaturen von 0 °C bis 200 °C, vorzugsweise von 40 °C bis 160 °C, behandelt.

Die hierbei intermediär gebildeten S-Acylmercaptophenylharnstoff der Formel (6) sind nur in Ausnahmefällen genügend hydrolysestabil, können aber bei absolut wasserfreiem Arbeiten isoliert werden. In der Regel werden sie unter Reaktionsbedingungen vom im Reaktionsgemisch enthaltenen oder nachfolgend zugesetzten Wasser in kurzer Zeit unter Abspaltung von Ammoniak und Kohlendioxid zu S-Acyl-o-aminothiophenolen der Formel (7) hydrolysiert, die, besonders rasch bei erhöhten Temperaturen, unter Wasserabspaltung zu den Zielverbindungen der genannten allgemeinen Formel (1) gemäß folgendem Reaktionsschema cyclisieren:

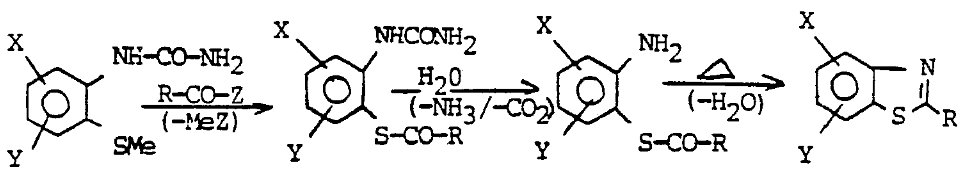


(5) (6) (7) (1)

Somit gelingt es nach dem erfindungsgemäßen Verfahren, die beispielsweise gemäß DE-PS 28 01 991 technisch leicht zugänglichen 2-Aminobenzthiazole der genannten Formel (4) in einer Eintopfreaktion durch Umsetzung mit einem Alkalimetall- oder Erdalkalimetallhydroxid und nachfolgend mit einem Acylierungsmittel der genannten Formel (3) in hoher Ausbeute und Reinheit in 2-substituierte Benzthiazole der genannten Formel (1) zu überführen. Das erfindungsgemäße Verfahren vermeidet den Einsatz freier Thiophenole und bietet daher keinerlei ökologische oder gewerbehygienische Probleme. Dadurch und in Verbindung mit der erzielbaren höheren Ausbeute stellt das erfindungsgemäße Verfahren einen erheblichen technischen Fortschritt dar.

Zudem überraschend ist, daß beim erfindungsgemäßen Verfahren keine merkliche Bildung von

Nebenprodukten beobachtet wird. Es war nämlich zu erwarten, daß beim Acylierungsschritt (in Gegenwart von Wasser), wenn auch nur in Spuren und örtlich begrenzt freiwerdende Säure den sehr leicht ablaufenden, sauer katalysierten Ringschluß des 2-Mercaptophenylharnstoffs zum entsprechenden 2-Hydroxybenzthiazol gemäß EP-PS 0 039 483 ermöglicht und dadurch durch 2-Hydroxybenzthiazol verunreinigte 2-substituierte Benzthiazole der genannten Formel (1) resultieren.

Daß diese erwarteten Nebenreaktionen beim erfindungsgemäßen Verfahren allenfalls in Spuren ablaufen und somit eine praktisch einheitliche Reaktion zu den gewünschten Zielprodukten der Formel (1) führt, war nicht vorauszusehen und erscheint auf Grund des komplexen Reaktionsgeschehens in mehreren Syntheseschritten äußerst überraschend.

In der EP-A-116 827 wird ein Verfahren zur Herstellung von Benzthiazolen beschrieben, wobei Amino- oder Nitrobenzole, die eine geeignete ortho-ständige Abgangsgruppe aufweisen, mit einem entsprechenden Thioamid oder Thioamid bildenden System umgesetzt werden. Ausbeuten und Reinheit der nach diesem Verfahren erhaltenen Benzthiazole sind unbefriedigend und dem Verfahren kommt nur eine geringe Anwendungsbreite zu, weshalb auch gegenüber dem durch diese Patentanmeldung gegebenen Stand der Technik ein Bedürfnis nach einem Verfahren, nach dem Benzthiazole in hohen Ausbeuten und hoher Reinheit hergestellt werden können, und dem eine größere Anwendungsbreite zukommt, bestand.

Das erfindungsgemäße Verfahren wird im einzelnen in der Weise durchgeführt, daß man das beispielsweise gemäß EP-PS 0 039 483 erhältliche 2-Mercaptophenylharnstoffsalz der allgemeinen Formel (5) als isolierten Feststoff oder in Form der beim Verdünnen des Ringöffnungsansatzes mit Wasser resultierenden wäßrigen Lösung (oder ggf. Suspension) bei Temperaturen von 0-200 °C, vorzugsweise von 40-160 °C, mit einem Acylierungsmittel der allgemeinen Formel (3), z. B. einer Carbonsäure, deren Halogenid oder Anhydrid, oder mit einem speziellen Carbonsäureanhydrid, wie beispielsweise Diketen, in mindest molarer Menge, vorzugsweise in einem Überschuß von 5 bis 25 %, innerhalb 0 bis 5, vorzugsweise 0,5 bis 2 Stunden versetzt, das gebildete 2-substituierte Benzthiazol durch Phasentrennung, Filtration oder Extraktion vom Reaktionsgemisch abtrennt und nachfolgend vom anhaftenden Wasser oder Extraktionsmittel befreit.

Selbstverständlich kann man auch von gemäß EP-PS 0 039 483 herstellbarem isoliertem freiem 2-Mercaptophenylharnstoff ausgehen, diesen nach Suspendieren in Wasser gegebenenfalls mit einer molaren Menge Alkalimetall- oder Erdalkalimetallhydroxid oder -carbonat zum vorstehend genannten Salz der allgemeinen Formel (5) neutralisieren und mit diesem in das Verfahren der Erfindung eintreten. Wegen der eingangs geschilderten Problematik freier Mercaptane stellt diese Variante jedoch keine bevorzugte dar.

Besonders bevorzugt ist dagegen die nachfolgende Variante, bei der ein aus einem isolierten 2-Mercaptophenylharnstoff-Salz (Formel (5) (vgl. EP-PS 0 039 483, Beispiele 6-10) und einem unter Reaktionsbedingungen gegenüber dem Acylierungsmittel der Formel (3) oder z. B. dem Diketen inerten Lösungsmittel (Toluol, Xylole, Chlorbenzol, Chlortoluole, Benzin, Chloraliphaten) hergestelltes Ausgangsgemisch oder ein isoliertes 2-Mercaptophenylharnstoff-Salz ohne Lösungsmittel mit dem Acylierungsmittel der Formel (3) oder z. B. mit dem Diketen bei Temperaturen von 0-200 °C, vorzugsweise 50 bis 160 °C in mindest molarer Menge, vorzugsweise in einem Überschuß von 5 bis 50 %, bei Abwesenheit eines Lösungsmittels mit einem gegebenenfalls erheblich höherem Überschuß, innerhalb von 1 bis 10, vorzugsweise 2 bis 6 Stunden, gegebenenfalls unter Druck, umgesetzt wird. Zur Aufarbeitung wird das Zielprodukt der Formel (1) durch Filtration, Auswaschen des Lösungsmittels und des gebildeten Salzes mit Wasser und Trocknen isoliert, oder, insbesondere bei flüssigen oder niedrigschmelzenden oder im Lösungsmittel zu gut löslichen Zielprodukten, gegebenenfalls nach Filtration vom gebildeten Salz, destillativ konzentriert und dann durch Filtration, Wäsche und Trocknung oder besonders vorteilhaft durch Fraktionierung, vorzugsweise im Vakuum von 0,1 mbar bis 135 mbar, in reiner Form gewonnen.

Die nachfolgenden Beispiele sollen das erfindungsgemäße Verfahren näher erläutern, ohne es zu beschränken. Die angegebenen Teile sind Gewichtsteile.

## Beispiel 1

Die gemäß Beispiel 3 der EP-PS 0 039 483 beim Umsatz von 82 Teilen 4-Methyl-2-aminobenzthiazol mit 50 Teilen Ätznatron erhaltene, mit Kohle geklärte Lösung des Natriumsalzes des 6-Methyl-2-mercaptophenylharnstoffes wird innerhalb von 15 Minuten unter Rühren bei 25 bis 30 °C mit 70 Teilen Essigsäureanhydrid versetzt. Anschließend läßt man 2 Stunden nachreagieren.

Man heizt dann zum Rückfluß, hält 1 Stunde den Rückfluß aufrecht und kühlt auf 0°C ab. Dabei granuliert das zunächst ölig abgeschiedene 2,4-Dimethylbenzthiazol der Formel

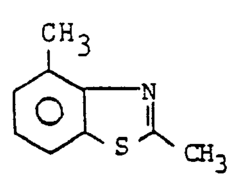

aus, welches abgesaugt, mit Wasser neutral gewaschen und im Vakuum bei Raumtemperatur getrocknet wird. Man erhält 75 Teile Produkt vom Ep. 33,5 °C, was einer Ausbeute von 92,0 % der Theorie, bezogen auf eingesetztes 4-Methyl-2-aminobenzthiazol, entspricht.

Das 2,4-Dimethylbenzthiazol ist in verdünnter Salzsäure klar löslich und zeigt im Gaschromatogramm (gemessen gegen inneren Standard) einen Reingehalt von 99,2 %. Der Reingehalt läßt sich auch durch potentiometrische Titration mit Perchlorsäure in Eisessig ermitteln und wird nach dieser Methode zu 98,9 % bestimmt.

Ersetzt man das Essigsäureanhydrid durch aliquote Mengen Acetylchlorid und arbeitet im übrigen in der angegebenen Weise, so erhält man ein vergleichbares Ergebnis.

Beispiel 2

Der gemäß EP-PS 0 039 483, Beispiel 1, Absatz 1 beim Umsatz von 150 Teilen 2-Aminobenzthiazol mit 150 Teilen Ätznatron nach wäßriger Aufarbeitung anfallende wasserfeuchte 2-Mercaptophenylharnstoff oder eine entsprechende Menge seines Natrium oder Kaliumsalzes wird in 500 Teilen 50 %iger Essigsäure gelöst und anschließend innerhalb von 2 Stunden bei 45 bis 50 °C tropfenweise mit 118 Teilen Diketen versetzt. Man rührt 1 Stunde nach, heizt dann zum Rückfluß und hält 2 Stunden eine Temperatur von 95 °C-100 °C. Man kühlt auf Raumtemperatur ab, isoliert das ausgefallene 2-Acetonylbenzthiazol der Formel

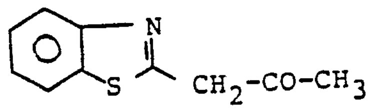

durch Filtration und trocknet im Vakuum bei 60 °C-80 °C. Man erhält 151,7 Teile Produkt vom Schmelzpunkt 118 °C-119 °C, was einer Ausbeute von 79,4 %, bezogen auf eingesetztes 2-Aminobenzthiazol, entspricht.

Das Produkt ist chromatografisch einheitlich und weist gemäß potentiometrischer Titration mit Perchlorsäure in Eisessig einen Reingehalt von 98,4 % auf.

Beispiel 3

85 Teile Natriumsalz des 2-Mercaptophenylharnstoffes (hergestellt gemäß Beispiel 1 der EP-PS 0 039 483 durch Umsetzung von 2-Aminobenzthiazol mit Ätznatron in Ethylenglykol bei 140 °C, anschließende Filtration der Reaktionsmischung und Trocknung des resultierenden Filterkuchens im Vakuum bei 100 °C) werden in 400 Teilen Chlorbenzol suspendiert.

Man gibt im Verlauf von 1 Stunde unter Rühren bei 60 °C-70 °C 69,5 Teile Phenylessigsäurechlorid zu, heizt dann auf 130 °C (Rückfluß) und hält 2 Stunden bei dieser Temperatur. Anschließend klärt man vom ausgefallenen Natriumchlorid, destilliert aus dem Filtrat ca. 250 Teile Chlorbenzol im Vakuum ab und kühlt den verbleibenden Sumpf auf Raumtemperatur ab. Das dabei ausfallende 2-Benzylbenzthiazol der Formel

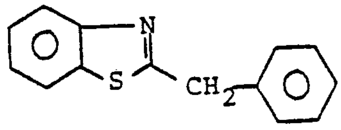

wird abgesaugt, mit wenig Chlorbenzol gewaschen und im Vakuum bei 60 °C-70 °C getrocknet.

Man erhält 102 Teile Produkt vom Schmelzpunkt 110 °C-111 °C, was einer Ausbeute von 90,7 % der Theorie, bezogen auf eingesetztes Natriumsalz, entspricht. Der durch potentiometrische Titration ($HClO_4$ in Eisessig) ermittelte Reingehalt liegt bei 99,1 %. Chromatografisch sind keine Verunreinigungen nachweisbar.

Beispiele 4-10

Ersetzt man in Beispiel 3 das Phenylessigsäurechlorid durch aliquote Teile der in der nachstehenden Tabelle 1 aufgeführten Acylierungsmittel (entsprechend der genannten allgemeinen Formel (3)) und arbeitet in der angegebenen Weise, wobei bei flüssigen Zielprodukten eine destillative Aufarbeitung durch Fraktionieren des vom Salz. befreiten Chlorbenzolfiltrates vorgenommen wird, so erhält man die aus der Tabelle 1 ersichtlichen 2-substituierten Benzthiazole (entsprechend der genannten allgemeinen Formel (1) mit X, Y = H) mit den angegebenen Schmelz- bzw. Siedepunkten, Ausbeuten und durch potentiometrische Titration ($HClO_4$ in Eisessig) ermittelten Reingehalten (RG) :

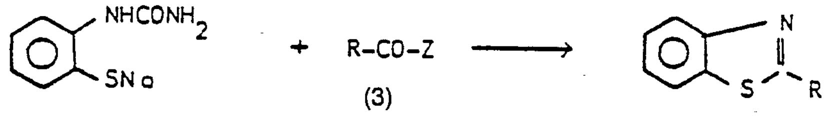

(3)

(Siehe Tabelle 1 Seite 7 f.)

Beispiel 11

112,25 Teile des gemäß Beispiel 6 der EP-PS 0 039 483 erhältlichen Natriumsalzes des 4-Chlor-2-mercaptophenylharnstoffes werden in 450 Teilen Eisessig in einem Autoklav suspendiert. Man verschließt das Druckgefäß und heizt in 2 Stunden langsam auf 150 °C-160 °C, wobei sich ein Druck von ca. 5 bar einstellt. Über ein Druckhalteventil wird gebildetes Wasser kontinuierlich abgeblasen. Nach 5 Stunden ist die Reaktion beendet. Man kühlt auf Raumtemperatur ab, öffnet den Autoklav, läßt das Reaktionsgemisch in 1 000 Teilen Wasser einlaufen und isoliert das dabei ausfallende 6-Chlor-2-methylbenzthiazol durch Filtration, wäscht neutral und trocknet im Vakuum bei 40 °C. Man erhält 85, 6 Teile Produkt der Formel

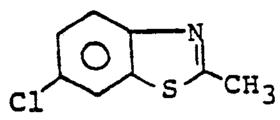

vom Schmelzpunkt 85 °C-86 °C, was einer Ausbeute von 93,3 % der Theorie, bezogen auf eingesetztes Natriumsalz, entspricht.

Die Verbindung ist chromatographisch einheitlich und zeigt bei potentiometrischer Titration mit $HClO_4$ in Eisessig einen Reingehalt von 98,9 %.

Beispiel 12-21

Ersetzt man in Beispiel 11 das Natriumsalz des 4-Chlor-2-mercaptophenylharnstoffes durch aliquote Mengen eines Salzes der allgemeinen Formel (8) und arbeitet sonst in der angegebenen Weise bei flüssigen Zielprodukten mit nachfolgender destillativer Aufarbeitung des Autoklavinhaltes durch Fraktionierung, so erhält man die in Tabelle 2 aufgeführten 2-Methylbenzthiazole der Formel (9) in den in der Tabelle angegebenen Ausbeuten und Reingehalten (potentiometrische Titration mit $HClO_4$ in Eisessig) und mit den dort niedergelegten Schmelz- bzw. Siedepunkten :

(Siehe Tabelle 2 Seite 8)

Beispiel 22

112,25 Teile des gemäß Beispiel 6 der EP-PS 0 039 483 erhältlichen, getrockneten Natriumsalzes des 4-Chlor-2-mercaptophenylharnstoffes werden in 350 Teilen o-Dichlorbenzol suspendiert. Man tropft bei 70 °C-80 °C unter Rühren 90 Teile Phenylessigsäurechlorid in 30 Minuten zu, heizt zum Sieden (ca. 165 °C) und hält 4 Stunden bei dieser Temperatur. Anschließend kühlt man auf 80 °C-90 °C, destilliert das Lösungsmittel durch Einleiten von Wasserdampf ab, läßt die erhaltene Emulsion auf Raumtemperatur unter Rühren abkühlen und isoliert das dabei ausgranulierende 6-Chlor-2-benzylbenzthiazol der Formel

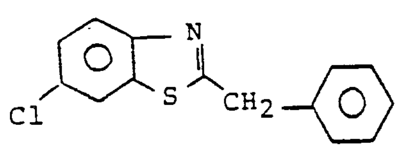

durch Filtration. Nach Waschen mit Wasser und Trocknen im Vakuum bei 40 °C-60 °C erhält man 121 Teile Produkt vom Schmelzpunkt 79 °C-81 °C, was einer Ausbeute von 93,3 % der Theorie, bezogen auf eingesetztes Natriumsalz, entspricht.

Die Verbindung ist chromatographisch einheitlich und ergibt bei potentiometrischer Titration mit Perchlorsäure einen Reingehalt von 98,2 %.

Beispiele 23-27

Ersetzt man im Beispiel 22 das Phenylessigsäurechlorid durch aliquote Mengen eines Säurechlorids der Formel (10) und das Natriumsalz des 4-Chlor-2-mercaptophenylharnstoffes durch entsprechende Mengen eines Natriumsalzes der Formel (11) und arbeitet im übrigen in der angegebenen Weise, so erhält man die in der nachstehenden Tabelle 3 aufgeführten 2-substituierter Benzthiazole der Formel (12) mit

Tabelle 1

| Beispiel | R | Z | Zielverbindung (1) | | | | |
| --- | --- | --- | --- | --- | --- | --- | --- |
| | | | R | Fp. °C | Kp. Mbar °C | Ausbeute | RG |
| 4 | $C_2H_5$ | $OCOC_2H_5$ | $C_2H_5$ | | Kp. 9.3 : 115° | 88.4 % | 98,7 % |
| 5 | $CH_2Cl$ | Cl | $CH_2Cl$ | | Kp. 19.9 : 145° | 86.9 % | 99.1 % |
| 6 | $n-C_4H_9$ | Br | $n-C_4H_9$ | | Kp. 9.3 : 132° | 92,4 % | 97,5 % |
| 7 | $t-C_4H_9$ | Cl | $t-C_4H_9$ | | Kp. 21.3 : 138° | 90,4 % | 98,0 % |
| 8 | 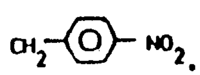 | Cl | 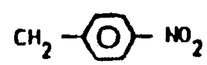 | 114° | - | 94,3 % | 97,8 % |
| 9 | 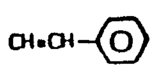 | Cl | 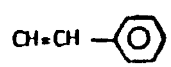 | 110-111° | - | 92,9 % | 98,9 % |
| 10 | 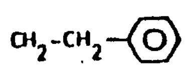 | Cl | 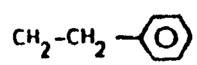 | 62° | - | 88,7 % | 98,9 % |

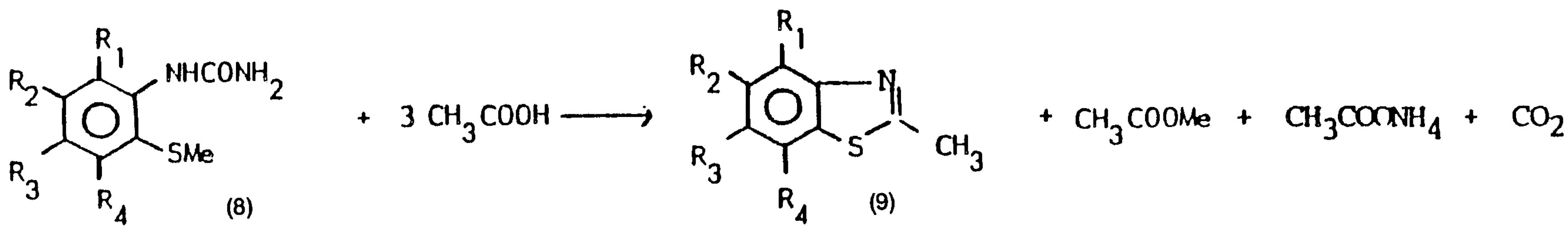


Tabelle 2

| Beispiel | Me | $R_1$ | $R_2$ | $R_3$ | $R_4$ | Zielverbindung (9) | | | |
|---|---|---|---|---|---|---|---|---|---|
| | | | | | | Fp. °C | Kp. (Torr) °C | Ausbeute | RG |
| 12 | Na | H | H | H | H | - | Kp. 19.9 : 115° | 86,4 % | 99,4 % |
| 13 | Mg/2 | Cl | H | H | H | 54° | - | 89,7 % | 99,2 % |
| 14 | Na | H | F | H | H | - | Kp. 9.3 : 112° | 85,9 % | 99,2 % |
| 15 | Na | H | H | $NO_2$ | H | 174° | - | 92,8 % | 98,8 % |
| 16 | K | H | H | $OCH_3$ | H | - | Kp. 33.3 : 176° | 81,8 % | 99,0 % |
| 17 | K | H | H | $OC_2H_5$ | H | 56° | - | 84,0 % | 98,3 % |
| 18 | Na | H | H | H | $CH_3$ | 31° | - | 86,9 % | 96,1 % |
| 19 | Na | Br | H | Br | H | 121° | - | 94,1 % | 98,4 % |
| 20 | Na | $CH_3$ | H | Cl | H | 78° | - | 93,8 % | 98,0 % |
| 21 | Na | - benzo - | | H | H | 95° | - | 90,8 % | 98,2 % |

den angegebenen Schmelzpunkten, Ausbeuten und Reingehalten :

(Siehe Tabelle 3 Seite 10 f.)

Beispiel 28

85 Teile Natriumsalz des 2-Mercaptophenylharnstoffes (hergestellt gemäß Beispiel 1 der EP-PS 0 039 483 durch Umsetzung von 2-Aminobenzthiazol mit Ätznatron in Ethylenglykol bei 140 °C, anschließende Filtration der Reaktionsmischung und Trocknen des resultierenden Filterkuchens im Vakuum bei 100 °C bis zur Gewichtskonstanz) werden in 250 Teilen o-Chlortoluol suspendiert. Man tropft unter Rühren bei 90 °C-100 °C 77 Teile Benzoylchlorid innerhalb 30 Minuten zu, heizt zum Sieden und kocht 5 Stunden unter Rückfluß. Anschließend destilliert man das o-Chlortoluol mit Wasserdampf ab, kühlt die resultierende wäßrige Emulsion bzw. Suspension auf Raumtemperatur ab und isoliert das ausgefallene 2-Phenylbenzthiazol der Formel

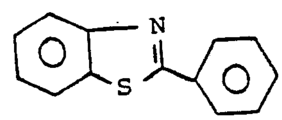

durch Filtration. Nach Neutralwaschen mit Wasser und Trocknen bei 80 °C im Umlaufschrank erhält man 100 Teile Produkt vom Schmelzpunkt 110 °C-112 °C, was einer Ausbeute von 94,8 % der Theorie, bezogen auf eingesetztes Natriumsalz, entspricht.

Das Produkt ist chromatographisch einheitlich. Sein durch potentiometrische Titration ($HClO_4$ in Eisessig) bestimmter Reingehalt liegt bei 98,5 %.

Beispiele 29-35

Ersetzt man in Beispiel 28 das Benzoylchlorid durch aliquote Mengen eines substituierten Benzoylchlorids der Formel (13) und arbeitet im übrigen in der angegebenen Weise, so erhält man die im Phenylrest substituierten 2-Phenylbenzthiazole der Formel (14) mit den in der nachstehenden Tabelle 4 aufgeführten Schmelzpunkten, Ausbeuten und Reingehalten :

(Siehe Tabelle 4 Seite 11 f.)

Beispiele 36-42

Ersezt man in Beispiel 28 das Natriumsalz des o-Mercaptophenylharnstoffes durch aliquote Mengen des Natriumsalzes eines substituierten o-Mercaptophenylharnstoffes der Formel (15) (herstellbar z. B. analog Beispiel 6 der EP-PS 0 039 483) und arbeitet im übrigen in der angegenenen Weise, so erhält man im Benzkern substituierte 2-Phenylbenzthiazole der Formel (16) mit den in der nachstehenden Tabelle 5 aufgeführten Schmelzpunkten, Ausbeuten und Reingehalten :

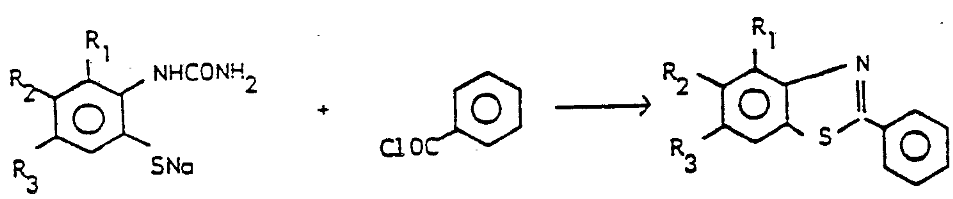

(15) (16)

(Siehe Tabelle 5 Seite 12 f.)

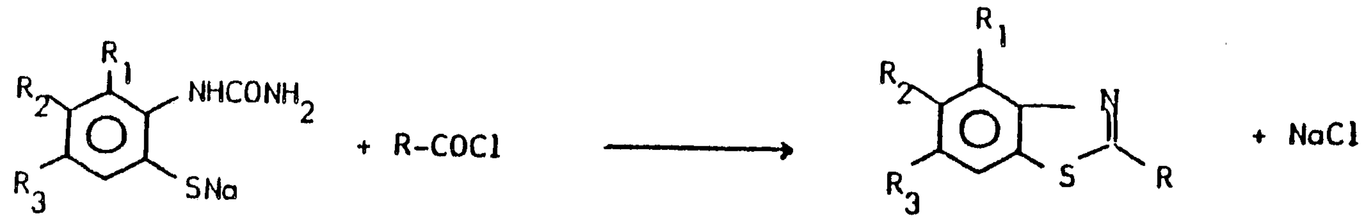


(11) (10) (12)

Tabelle 3

| Beispiel | Edukte (10,'11) | | | | Produkt (12) | | |
|---|---|---|---|---|---|---|---|
| | R | $R_1$ | $R_2$ | $R_3$ | Fp. °C | Ausbeute | RG |
| 23 | $CH_3$ | H | H | Br | 86-87° | 94,3 % | 98,8 % |
| 24 | $CH_3$ | H | $NO_2$ | H | 136-138° | 91,0 % | 97,5 % |
| 25 | $C_2H_5$ | H | Cl | H | 55-57° | 89,5 % | 97,2 % |
| 26 | CH=CH- 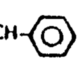 | Cl | H | Cl | 152-154° | 92,8 % | 97,5 % |
| 27 | CH=CH- 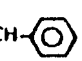 | H | H | Cl | 129-130° | 95,1 % | 99,2 % |

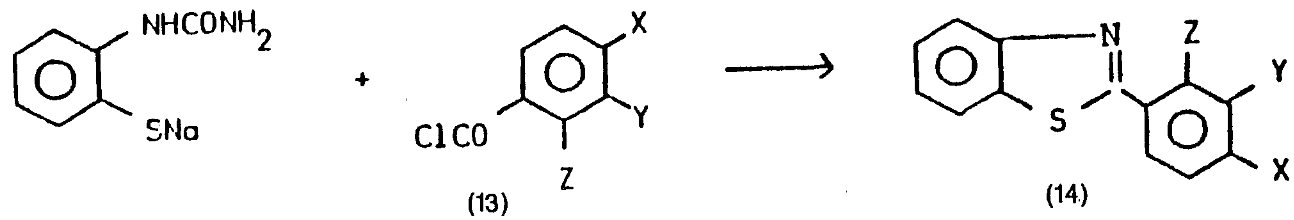

(13) (14)

Tabelle 4

| Beispiel | Edukt (13) | | | Produkt (14) | | |
|---|---|---|---|---|---|---|
| | X | Y | Z | Fp °C | Ausbeute | RG |
| 29 | $CH_3$ | H | H | 83-84° | 91,5 % | 98,5 % |
| 30 | $OCH_3$ | H | H | 132-135° | 90,8 % | 97,2 % |
| 31 | Cl | H | H | 110-112° | 95,8 % | 99,0 % |
| 32 | $NO_2$ | H | H | 232-233° | 96,5 % | 98,4 % |
| 33 | Cl | H | Cl | 143-145° | 96,2 % | 99,1 % |
| 34 | H | $NO_2$ | H | 184-185° | 92,4 % | 97,2 % |
| 35 | H | H | $NO_2$ | 111-114° | 90,8 % | 97,5 % |

Tabelle 5

| Beispiel | Edukt (15) | | | Produkt (16) | | |
|---|---|---|---|---|---|---|
| | $R_1$ | $R_2$ | $R_3$ | Schmelzpunkt °C | Ausbeute | RG |
| 36 | Br | H | Br | 165 - 168° | 94,4 % | 97,9 % |
| 37 | H | $CH_3$ | H | 147 - 148° | 91,5 % | 99,0 % |
| 38 | H | $NO_2$ | H | 191 - 192° | 95,8 % | 97,5 % |
| 39 | H | H | $CH_3$ | 120 - 122° | 89,8 % | 99,2 % |
| 40 | H | H | $OCH_3$ | 114 - 115° | 87,8 % | 98,1 % |
| 41 | H | H | Cl | 155 - 156° | 93,5 % | 98,9 % |
| 42 | H | H | $NO_2$ | 190 - 192 | 96,0 % | 98,1 % |

Beispiele 43-49

Ersetzt man in Beispiel 28 das Natriumsalz des o-Mercaptophenylharnstoffes durch aliquote Mengen des Natriumsalzes eines substituierten o-Mercaptophenylharnstoffes der Formel (17) (herstellbar z. B. analog Beispiel 6 der EP-PS 0 039 483) und das Benzoylchlorid durch entsprechende Mengen eines substituierten Benzoylchlorids der Formel (13) und arbeitet im übrigen in der angegebenen Weise, so erhält man die in der nachstehenden Tabelle 6 aufgeführten, im Benz- und Phenylkern substituierten 2-Phenylbenzthiazole der Formel (18) mit den angegebenen Schmelzpunkten, Ausbeuten und Reingehalten :

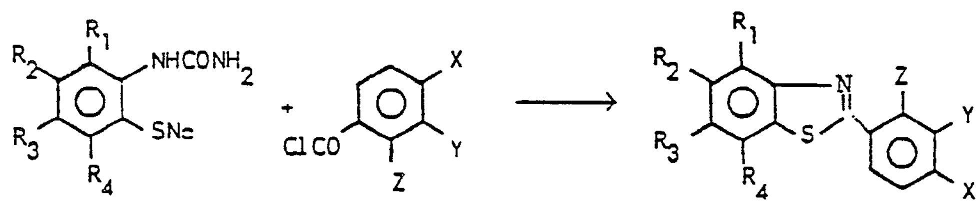

(17) (13) (18)

(Siehe Tabelle 6 Seite 13 f.)

Tabelle 6

| Beispiel | Edukt (17) | | | | Edukt (13) | | | Produkt (10) | | |
|---|---|---|---|---|---|---|---|---|---|---|
| | $R_1$ | $R_2$ | $R_3$ | $R_4$ | X | Y | Z | Schmelzpunkt °C | Ausbeute | RG |
| 43 | $CH_3$ | H | $CH_3$ | H | H | $CH_3$ | H | 71-74° | 85,2 % | 96,8 % |
| 44 | H | $CH_3$ | H | H | H | $NO_2$ | H | 184-185° | 96,5 % | 99,1 % |
| 45 | H | Cl | H | H | H | H | $NO_2$ | 195-197° | 96,8 % | 98,9 % |
| 46 | H | H | $CH_3$ | H | $OC_2H_5$ | H | H | 167-169° | 87,8 % | 96,2 % |
| 47 | H | H | Br | H | Br | H | H | 215-218° | 97,4 % | 98,0 % |
| 48 | H | H | $NO_2$ | H | $NO_2$ | H | H | 238-240° | 97,5 % | 97,5 % |
| 49 | H | H | H | $CH_3$ | H | $NO_2$ | H | 179-180° | 91,4% | 97,2 % |

**Patentanspruch**

Verfahren zur Herstellung von Benzthiazolen der allgemeinen Formel (1)

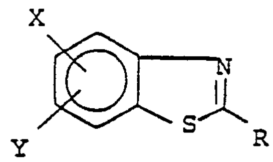

(1)

in welcher R eine Alkyl($C_1$-$C_6$)- oder Alkenyl($C_2$-$C_6$)-gruppe, welche durch Alkoxy($C_1$-$C_4$), Acylgruppen, Phenyl-, Chlorphenyl-, Bromphenyl-, Alkyl($C_1$-$C_4$)-phenyl-, Alkoxy($C_1$-$C_4$)-phenyl- oder Nitrophenylgruppen oder Halogenatome substituiert sein können, oder eine Phenylgruppe, die durch Alkyl($C_1$-$C_4$), Alkoxy($C_1$-$C_4$), Carboxyl-, —COO—Alkyl($C_1$-$C_4$)-, Cyan- oder Nitrogruppen oder durch Halogentome substituiert sein kann, bedeutet, X und Y je ein Wasserstoff- oder Halogenatom, oder eine Alkyl($C_1$-$C_4$)-, Alkoxy($C_1$-$C_4$)- oder Nitrogruppe oder zusammen einen anellierten Benzolring bedeuten, dadurch gekennzeichnet, daß man die in bekannter Weise durch Umsetzung von 2-Aminobenzthiazolen der allgemeinen Formel (4)

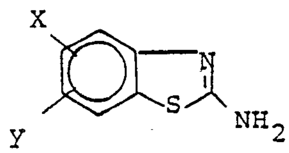

(4)

in welcher X und Y die vorstehend genannten Bedeutungen haben, mit Alkalimetall- oder Erdalkalimetallhydroxiden in einem wasserfreien oder praktisch wasserfreien Lösemittel als Zwischenverbindungen erhältlichen Salze der entsprechenden 2-Mercaptophenylharnstoffe der allgemeinen Formel (5)

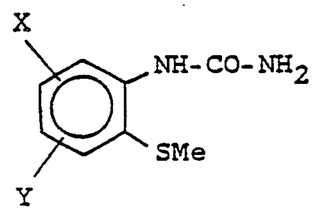

(5)

in welcher X und Y die genannten Bedeutungen haben, und Me ein Alkalimetallatom oder die äquivalente Menge eines Erdalkalimetallatoms bedeutet, in isolierter Form oder in der im Ringöffnungsgemisch vorliegenden Suspension mit einem Acylierungsmittel der allgemeinen Formel (3)

$$R—CO—Z \qquad (3)$$

in welcher R die vorstehend genannte Bedeutung hat und Z ein Halogenatom, die Hydroxylgruppe oder die Gruppe

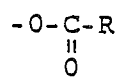

,

worin R die genannte Bedeutung hat, darstellt, oder mit Diketen bei Temperaturen von 0°C bis 200 °C behandelt.

**Claim**

A process for the preparation of benzothiazoles of the general formula (1)

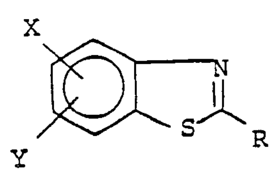

(1)

in which R denotes an alkyl($C_1$-$C_6$) or alkenyl($C_2$-$C_6$) group which can be substituted by alkoxy($C_1$-$C_4$), acyl groups, phenyl, chlorophenyl, bromophenyl, alkyl($C_1$-$C_4$) phenyl, alkoxy($C_1$-$C_4$) phenyl or nitrophenyl groups or halogen atoms or denotes a phenyl group which can be substituted by alkyl($C_1$-$C_4$), alkoxy($C_1$-$C_4$), carboxyl, —COO—alkyl($C_1$-$C_4$) cyano or nitro groups or by halogen atoms, and X and Y each denote a hydrogen or halogen atom, or an alkyl($C_1$-$C_4$), alkoxy($C_1$-$C_4$) or nitro group, or together denote a fused benzene ring, wherein the salts of the corresponding 2-mercaptophenylureas of the general formula (5),

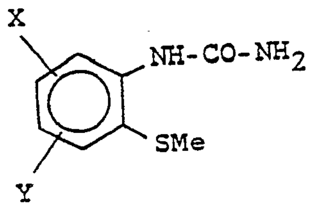

(5)

in which X and Y have the stated meanings and Me denotes an alkali metal atom or the equivalent amount of an alkaline earth metal atom, which salts are obtainable as intermediate compounds, in a known manner by reacting 2-aminobenzothiazoles of the general formula (4)

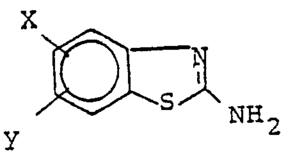

(4)

in which X and Y have the abovementioned meanings, with alkali metal or alkaline earth metal hydroxides in an anhydrous or virtually anhydrous solvent, are treated, in the isolated form or in suspension in the ring-opening mixture, with an acylating agent of the general formula (3)

$$R—CO—Z \qquad (3)$$

in which R has the abovementioned meaning and Z represents a halogen atom, the hydroxyl group or the group

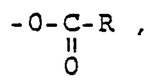

wherein R has the stated meaning, or with diketene at temperatures from 0 °C to 200 °C.

**Revendication**

Procédé pour préparer des benzothiazoles de formule générale (1)

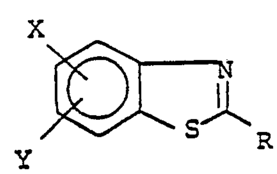

(1)

dans laquelle R représente un groupe alkyle en $C_1$ à $C_6$ ou alcényle en $C_2$ à $C_6$ (pouvant être substitués par un groupe alcoxy en $C_1$ à $C_4$, par des groupes acyles, par des groupes phényle, chlorophényle, bromophényle, alkyl(en $C_1$ à $C_4$)-phényle, alcoxy(en $C_1$ à $C_4$)-phényle ou nitrophényle ou par des atomes d'halogène) ou un groupe phényle (pouvant être substitué par un groupe alkyle en $C_1$ à $C_4$, alcoxy en $C_1$ à $C_4$, carboxyle, —COO—alkyle en $C_1$ à $C_4$, cyano ou nitro, ou par des atomes d'halogène ; X et Y représentent chacun un atome d'hydrogène ou d'halogène ou un groupe alkyle en $C_1$ à $C_4$, alcoxy en $C_1$ à $C_4$ ou nitro, ou bien ils forment ensemble un noyau benzénique condensé, procédé caractérisé en ce qu'on soumet les sels des mercapto-2 phénylurées correspondantes, de formule générale (5) :

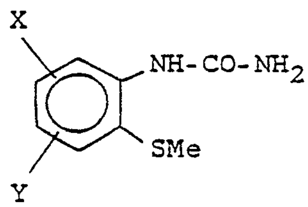

(5)

(dans laquelle X et Y ont les sens indiqués, et Me représente un atome de métal alcalin ou la quantité équivalente d'un atome de métal alcalino-terreux), celles que l'on peut obtenir de façon connue par réaction d'amino-2 benzothiazoles de formule générale (4)

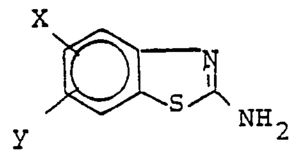

(4)

(dans laquelle X et Y ont les sens précités) avec des hydroxydes de métaux alcalins ou de métaux alcalino-terreux, dans un solvant anhydre ou pratiquement anhydre, à titre de composés intermédiaires, sous forme isolée ou dans la suspension présente dans le mélange d'ouverture de cycle, à un traitement avec un agent d'acylation de formule (3) générale :

$$R—CO—Z \qquad (3)$$

dans laquelle R a le sens précité et Z représente un atome d'halogène, le groupe hydroxyle ou le groupe

$$-O-\underset{\underset{O}{\|}}{C}-R \ ,$$

(où R a le sens précité), ou bien on traite par du dicétène à des températures de 0 °C jusqu'à 200 °C.